# EUROPEAN PATENT SPECIFICATION

(11) **EP 2 374 497 B1**
(45) Date of publication and mention of the grant of the patent: **19.10.2016**
(21) Application number: 10159337.4
(22) Date of filing: 08.04.2010
(51) Int. Cl.: A61M 39/20, A61M 5/32

(54) **Syringe tip cap having fluoropolymer coated insert**
Spritzenspitzenkappe mit fluorpolymerbeschichtetem Einsatz
Capuchon de pointe de seringue dotée d'un insert revêtu de fluoropolymère

(43) Date of publication of application: 12.10.2011
(73) Proprietor: West Pharmaceutical Services Deutschland GmbH & Co. KG, 52249 Eschweiler (DE)
(72) Inventor: Grün, Heike, 52074, Aachen (DE); Brinkhues, Jürgen, 52072, Aachen (DE); Krautgartner, Klaus, 52351, Düren (DE); Wawzik, Joachim, 52134, Herzogenrath (DE)
(74) Representative: Lippert, Stachow & Partner

(56) References cited:
- WO-A1-96/37247
- WO-A1-03/090825
- WO-A1-2005/032627
- WO-A1-2009/143439
- DE-U1- 8 906 101
- US-A1- 2003 078 546
- US-A1- 2007 156 100
- US-A1- 2007 255 237
- US-B1- 6 485 474

## Description

### Field of the Invention

The present invention relates to a tip cap for use with a syringe, the process for making said tip cap and also to a prefilled syringe comprising said tip cap. Specifically, the invention relates to a syringe tip cap comprising - a body having two end faces, wherein one of the end faces is provided with an opening leading to an axial blind bore having a bottom and an inner side wall, and an insert consisting of elastic material fitted into the bore and having two faces, wherein one of the faces abuts to the bottom of the bore and the other is exposed to the bore.

### Background of the Invention

Prefilled syringes containing various drugs and medicaments for use in medical treatment of humans and animals are widely known. These products facilitate the administration and the dosage of the medicaments and are therefore frequently used. If the medicament itself is stable, prefilled syringes may have a shelf life of one year or significantly more, provided that there is no adverse effect from the material of the different parts of the syringe or from the atmosphere through leaks in the containment.

A normal syringe has a barrel from glass or plastic and a displaceable plunger which forms a tight seal with the inner surface of the barrel. The end face of the barrel opposite to the plunger is provided with means for connecting the syringe to hypodermic needles, infusion tubing and the like. This is mostly a tip in the form of a so called luer cone or luer taper. Prior to use of the prefilled syringe the passage in this connection is usually sealed with a so called tip cap in order to avoid leakage of the contents and access of atmospheric components such as oxygen. Such a tip cap comprises a body having two end faces, wherein one of the end faces is provided with an opening leading to an axial blind bore having a bottom. This blind bore may be cylindrical or slightly tapered, i. e. narrowing from the opening to the bottom so as to approximate the shape of the luer cone. To seal the opening in the tip after filling the syringe barrel, the syringe tip is introduced into the blind bore of the tip cap until its opening is pressed against the bottom of the bore, whereby the opening is sealed.

This tip cap has to meet two major requirements. It has to be sufficiently elastic and resilient to fit tightly to the tip and to compensate for unavoidable deviations in shape and dimension of the tip, such as dimples in the surface or deviation from the circular shape. On the other hand, since the material of the tip cap may contact the contents of the syringe via the passage in the tip, this material must not release any components which may pollute this contents or react with it.

WO-A1-2005/032627 discloses a syringe tip cap made from a specific polymer composition which can be sterilized without loss of flexibility and resilience and has low extractables after sterilization. DE 89006101 discloses a so-called rigid needle shield with an outer rigid structure, namely the body, into which an inner softer member is inserted. Such rigid needle shields are also disclosed in WO 2009/143429, US 2007/0255237 and US 2003/0078546.
the cap and the outside of the tip so that the cap may tend to slide off the tip unintentionally. Moreover the fluoropolymer film is stiffer than the rubber itself and may therefore not fit completely to the tip surface in case of dimples or other irregularities, thereby reducing adhesion further and in the worst case causing leaks.

### Summary of the Invention

Therefore it is the problem underlying the present invention to provide a syringe tip cap which avoids the aforementioned problems of the prior art.

This problem is solved by a tip cap in accordance with claim 1. It has been found, that tight fit of the cap to the syringe tip and compatibility of the tip with the contents of the syringe can be achieved concurrently if the rubber body of the cap is provided with an insert consisting of elastic material fitted into the bore and having two end faces, wherein one end face abuts to the bottom of the bore and the other is exposed to the bore, wherein the surface of the insert is coated with a fluoropolymer film at least on the exposed end face. The elastomer inner side wall gives a tight and stable fit of the cap to the tip, whereas the fluoropolymer film at the exposed end face of the insert is pressed to the opening and provides a tight seal resistant to the contents of the syringe.

The elastic material in the body and the insert is natural or synthetic rubber, particularly natural rubber, butyl rubber, chlorobutyl rubber or bromobutyl rubber. This rubber is vulcanised as usual.

It is possible to form the body and the insert from the same material. In another preferred embodiment the materials may be different having different elastic properties. This way it is possible to optimise the elastic properties of the body for tight and solid fit to the syringe tip, whereas the material is chosen for optimum sealing of the fluoropolymer film to the tip opening.

Any fluoropolymer which is available in form of film can be used, e. g. PTFE (polytetrafluoroethylene), FEP (fluoroethylene-propylene copolymer), PFA (perfluoroalkoxypolyethylene), ETFE (ethylene-tetrafluoroethylene copolymer), PVF (polyvinylfluoride), PCTFE (polychlorotrifluoroethylene), ECTFE (polyethylene-chlorotrifluoroethylene). Preferred materials are PTFE, FEP, and ETFE.

The fluoropolymer film has preferably a thickness of 0.02 to 0.5 mm.

In a preferred embodiment the insert has a protrusion on the end face coated with the fluoropolymer film, which is arranged so that it is capable of meeting the orifice after assembly of the syringe and tip. This way the sealing effect is improved.

The dimensions of the insert relative to the body are preferably chosen so that the insert is held within the blind bore of the body and will not easily fall out. Since both body and insert are made from elastic material, it is possible to make the outer diameter of the insert somewhat bigger than the inner diameter of the blind bore. After introduction of the insert into the blind bore the insert is then held by kind of a press fit. Suitably the insert is 0.2 to 1.0 mm bigger than the blind bore.

The axial length of the insert may preferably be in the region between about one third and three times the diameter of the insert.

The outer contour of the tip cap may have additional features like a thread, blade or the like to enable a fit to a luer lock for assisting fixation of the cap to the syringe cone.

Methods for manufacturing a tip cap according to the invention are generally known from general technology of making rubber parts, e. g. compression molding, compression or injection transfer molding, pad molding, powder injection molding. The compound mixture comprising the uncured rubber or other elastomer, the curing agent and other additives is placed or injected into a mould and kept at elevated temperature and pressure for a time sufficient for vulcanization to take place. This molding procedure can be applied for making the cap body as well as the insert. After molding the insert, the fluoropolymer film can be coated onto the surface thereof. Alternatively, a blank of fluoropolymer film is placed in the mold for molding the insert before feeding the raw elastic material into the mold. The fluoropolymer film, being placed in the mold in advance, is secured to the insert during vulcanization. It is however preferred to prepare a compact of the compound mixture in a suitable shape and to place it in the mold together with a blank of the fluoropolymer film.

Assembly of the tip cap according to the invention can be accomplished by pressing the insert manually into the blind bore of the body or by using mechanically driven devices. For assembly the parts can be suitably held by vacuum holders. It is also possible to make the insert first and to place this in the mold when molding the body. This would save an extra assembly step.

The tip cap according to the invention withstands thermal sterilization without deterioration of its sealing properties.

### Brief Description of the Drawings

Figure 1 is an axial section of a syringe tip cap of the prior art as described above,
Figure 2 is an axial section of a syringe tip cap according to the invention,

### Detailed Description of the Invention

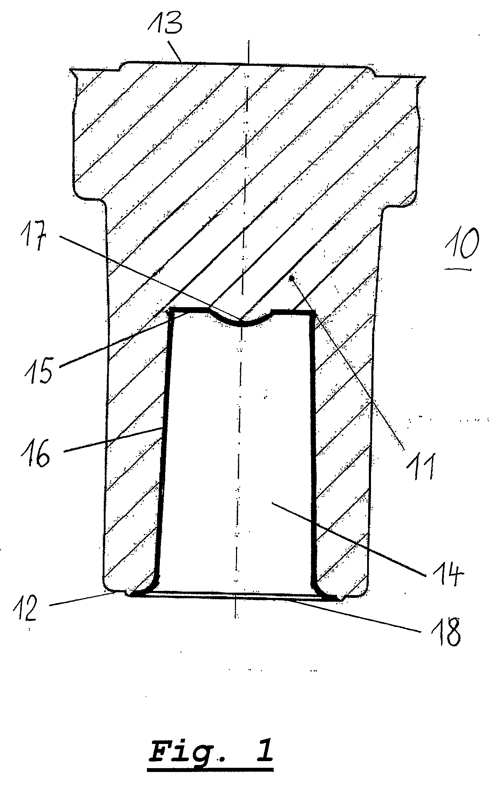

Fig. 1 shows a syringe tip cap 10 of the prior art. It is generally cylindrical and comprises two end faces 12, 13. End face 12 is provided with an opening 18, which leads to a somewhat tapered blind bore 14. Bore 14 ends at bottom 15, which is provided with a protrusion 17. The side walls, bottom and protrusion are coated with a fluoropolymer film 16. In use the tip cap is put over the syringe tip so that the fluoropolymer film 16 comes into contact with the surface of the tip cone. The protrusion 17 is pressed against the orifice of the syringe tip. Since the adhesion and friction between the fluoropolymer film 16 and the material of the tip (glass or plastic) may be low, there is danger that the cap may slide off the tip.

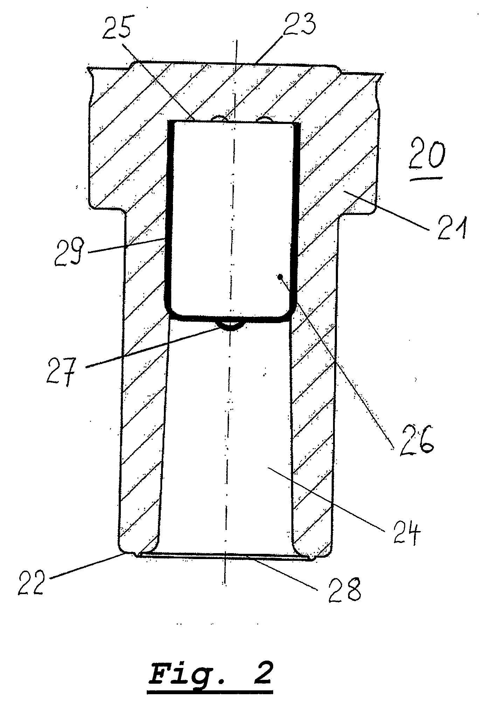

Fig. 2 is an axial section of a tip cap 20 according to the present invention. Its body 21 is again generally cylindrical with two end faces 22, 23. A somewhat tapered blind bore 24 extends from opening 28 in end face 22 and ends at bottom 25. An insert 26 is introduced into blind bore 24 and abuts with one end face against bottom 25. The exposed end face of the insert 26 and the side surface thereof are coated with fluoropolymer film 29. This film covers also protrusion 27 in the center of the exposed end face. The diameter of the insert 26 in this example is somewhat bigger than the inner diameter of bore 24. Therefore bore 24 must be elastically widened when insert 26 is introduced. Film 29 on the side surface lowers friction and facilitates introduction. However it would be possible to coat only the exposed end face of the insert with the fluoropolymer film.

In use the cap is put on the syringe tip so that the inner side wall of blind bore 24 comes into contact with the glass or plastic surface of the tip. Since both adhesion and friction of rubber against glass or plastic is much higher than those of fluoropolymer of prior art, the tip cap is securely held and can not slide off. Moreover the resilient inner rubber surface of bore 24 compensates any unevenness in the tip surface and provides continuous contact between tip and cap, thus improving fixation of the cap on the tip. On the other hand, the orifice of the tip is securely closed by protrusion 27 on the exposed end face by fluoropolymer film 29 with no danger of loosening and contamination of the contents of the syringe.

## Claims

1. A syringe tip cap (20) comprising
- a body having two end faces (22, 23) , wherein one of the end faces (22) is provided with an opening (28) leading to an axial blind bore (24) having a bottom (25) and an inner side wall, and
- an insert (26) consisting of elastic material fitted into the bore and having two end faces, wherein one end face abuts to the bottom (25) of the bore and the other is exposed to the bore (24), **characterized in that**
- a surface of the insert is coated with a fluoropolymer film (29) at least on the exposed end face,
- said body consisting of an elastic material designed to give a tight and stable fit of the cap to a syringe tip,
- the elastic material is natural or synthetic rubber,
- the fluoropolymer film (29) comprises polytetrafluorethylene, fluoroethylene-propylene copolymer or ethylene-tetrafluoroethylene copolymer,
- the elastic material of the body is different from that of the insert,
- and **in that** the the fluoropolymer film (29) at the exposed end face of the insert is configured to be pressed to an opening of a syringe tip to provide a tight seal resistant to the contents of the syringe.

2. Tip cap according to claim 1, wherein the synthetic rubber is butyl rubber, chlorobutyl rubber or bromobutyl rubber.

3. Tip cap according to one of the preceding claims, wherein the fluoropolymer film has a thickness of 0,02 to 0,5 mm.

4. Tip cap according to one of the preceding claims, wherein the end face coated with the fluoropolymer film has a protrusion capable of meeting the orifice of the syringe tip.

5. Tip cap according to one of the preceding claims, wherein before assembly the outer diameter of the insert is bigger than the inner diameter of the blind bore.

6. Tip cap according to one of the preceding claims, wherein the side surface of the insert is also coated with fluoropolymer film.

7. A method for manufacturing a syringe tip cap (20) according to one of the preceding claims, comprising the steps
- making a body (21) having two end faces (22,23), wherein one of the end faces (22) is provided with an opening (28) leading to an axial blind bore (24) having a bottom (25) and an inner side wall, said body consisting of an elastic material, by a thermal molding process,
- making an insert (26) consisting of elastic material having two end faces by a thermal molding process, the elastic material of the body is different from that of the insert and the material of the body is designed to give a tight and stable fit of the cap to the tip,
- wherein the elastic material is natural or synthetic rubber,
- coating at least one of the end faces of the insert (26) with a fluoropolymer film (29),
- wherein the fluoropolymer film comprises polytetrafluorethylene, fluoroethylene-propylene copolymer or ethylene-tetrafluoroethylene copolymer,
- assembling the tip cap (20) by introducing the insert into the blind bore so that the insert end face coated with fluoropolymer is exposed to the bore (24) and the other end abuts to the bottom (25) of the bore (24).

8. Method according to claim 7, wherein the thermal molding process is compression molding, compression or injection transfer molding, pad molding or powder injection molding.

9. Method according to one of claims 7 or 8, wherein a curing agent is added to the elastic material to vulcanize the material during thermal molding.

10. Method according to one of claims 7 to 9, wherein a blank of fluoropolymer film (29) is placed in the mold before molding the insert (26).

## Patentansprüche

1. Spritzenspitzenkappe (20), umfassend
- einen Körper mit zwei Endflächen (22, 23), wovon eine Endfläche (22) mit einer Öffnung (28) versehen ist, die zu einer axialen Blindbohrung (24) führt, die einen Boden (25) und eine innere Seitenwand hat, und
- einen Einsatz (26) aus einem elastischen Material, der in die Bohrung eingesetzt ist und zwei Endflächen hat, wobei eine der Endflächen an dem Boden (25) der Bohrung anliegt und die andere zur Bohrung (24) exponiert ist, **dadurch gekennzeichnet,**
- **dass** eine Oberfläche des Einsatzes zumindest an der exponierten Endfläche mit einem Fluorpolymerfilm (29) beschichtet ist,
- **dass** der aus einem elastischen Material bestehende Körper derart ausgebildet ist, dass dieser für einen dichten und stabilen Sitz der Kappe an einer Spritzenspitze sorgt,
- **dass** das elastische Material Naturkautschuk oder synthetischer Kautschuk ist,
- **dass** der Fluorpolymerfilm (29) Polytetrafluorethylen, Fluorethylenpropylen-Copolymer oder Ethylen-Tetrafluorethylen-Copolymer umfasst,
- **dass** sich das Material des Körpers von jenem des Einsatzes unterscheidet und
- **dass** der Fluorpolymerfilm (29) an der exponierten Endfläche des Einsatzes derart konfiguriert ist, dass dieser an eine Öffnung einer Spritzenspitze gedrückt wird, um für eine sichere Abdichtung gegenüber dem Inhalt der Spritze zu sorgen.

2. Spitzenkappe nach Anspruch 1, wobei der synthetische Kautschuk, Butylkautschuk, Chlorbutylkautschuk oder Brombutylkautschuk ist.

3. Spitzenkappe nach einem der vorhergehenden Ansprüche, wobei der Fluorpolymerfilm eine Dicke von 0,02 bis 0,5 mm aufweist.

4. Spitzenkappe nach einem der vorhergehenden Ansprüche, wobei die mit dem Fluorpolymerfilm beschichtete Endfläche einen Vorsprung hat, der geeignet ist, die Öffnung der Spritzenspitze zu treffen.

5. Spitzenkappe nach einem der vorhergehenden Ansprüche, wobei vor der Montage der Außendurchmesser des Einsatzes größer ist als der Innendurchmesser der Blindbohrung.

6. Spitzenkappe nach einem der vorhergehenden Ansprüche, wobei die Seitenfläche des Einsatzes ebenfalls mit einem Fluorpolymerfilm beschichtet ist.

7. Verfahren zum Herstellen einer Spritzenspitzenkappe (20) nach einem der vorhergehenden Ansprüche, umfassend die Schritte:
- Herstellen eines Körpers (21) mit zwei Endflächen (22, 23), wovon die eine Endfläche (22) mit einer Öffnung (28) versehen ist, die zu einer axialen Blindbohrung (24) führt, die einen Boden (25) und eine innere Seitenwand hat, wobei der Körper aus einem elastischen Material besteht, durch ein Thermoformverfahren,
- Herstellen eines aus einem elastischen Material bestehenden Einsatzes (29) mit zwei Endflächen durch ein Thermoformverfahren, wobei sich das elastische Material des Körpers von jenem des Einsatzes unterscheidet und wobei das Material des Körpers derart ausgebildet ist, dass dieses für einen dichten und stabilen Sitz der Kappe an der Spitze sorgt,
- wobei das elastische Material Naturkautschuk oder synthetischer Kautschuk ist,
- das Beschichten mindestens einer der Endflächen des Einsatzes (26) mit einem Fluorpolymerfilm (29),
- wobei der Fluorpolymerfilm Polytetrafluorethylen, Fluorethylenpropylen-Copolymer oder Ethylen-Tetrafluorethylen-Copolymer umfasst,
- Montieren der Spitzenkappe (20) durch Einführen des Einsatzes in die Blindbohrung, so dass die mit Fluorpolymer beschichtete Endfläche des Einsatzes zur Bohrung (24) exponiert ist und das andere Ende an dem Boden (25) der Bohrung (24) anliegt.

8. Verfahren nach Anspruch 7, wobei das Thermoformverfahren ein Formpressverfahren, ein Form- oder Spritzpressverfahren, ein Pad-Formungsverfahren oder ein Pulver-Spritzgussverfahren ist.

9. Verfahren nach einem der Ansprüche 7 oder 8, wobei dem elastischen Material ein Härtungsmittel zugegeben wird, um das Material während der Thermoformung zu vulkanisieren.

10. Verfahren nach einem der Ansprüche 7 bis 9, wobei ein Zuschnitt eines Fluorpolymerfilms (29) vor dem Formen des Einsatzes (26) in der Form angeordnet wird.

## Revendications

1. Capuchon de pointe de seringue (20), comprenant:
- un corps avec deux faces d'extrémité (22, 23), l'une desdites faces d'extrémité (22) étant prévue d'un orifice (28) débouchant sur un alésage borne axial (24) comportant un fond (25) et une paroi latérale interne, et
- un insert (26) qui consiste en un matériau élastique installé dans ledit alésage et comportant deux faces d'extrémité, l'une desdites faces venant en butée contre le fond (25) de l'alésage et l'autre étant exposée à l'alésage (24), **caractérisé en ce que**
- une surface de l'insert est revêtue d'un film fluoropolymère (29) au moins sur la face d'extrémité exposée,
- ledit corps consistant en un matériau élastique est conçu pour assurer une ajustage étroite et stable du capuchon sur le pointe de seringue,
- le matériau élastique est caoutchouc naturel ou synthétique,
- le film fluoropolymère (29) comprend de polytétrafluoroéthylène, un copolymère de fluoroéthylènepropylène ou un copolymère d'éthylènetetrafluoroéthylène,
- le matériau élastique du corps est différent de celui-ci de l'insert, et
- le film fluoropolymère (29) sur la face d'extrémité exposée de l'insert est configurer pour être préssée contre un orifice d'une pointe de seringue afin de former un joint étanche résistant au contenu de la seringue.

2. Capuchon de pointe selon la revendication 1, dans lequel le caoutchouc synthétique est un caoutchouc butyle, un caoutchouc chlorobutyle ou un caoutchouc bromobutyle.

3. Capuchon selon l'une des revendications précédentes, dans lequel le film fluoropolymère a une épaisseur de 0,02 à 0,5 mm.

4. Capuchon selon l'une des revendications précédentes, dans lequel la face d'extrémité revêtue du film fluoropolymère présente une saille capable de rencontrer l'orifice de la pointe de seringue.

5. Capuchon selon l'une des revendications précédentes, dans lequel avant l'assemblage, le diamètre extérieur de l'insert est plus grand que le diamètre intérieur de l'orifice borne.

6. Capuchon selon l'une des revendications précédentes, dans lequel la face latérale de l'insert est également revêtue d'un film fluoropolymère.

7. Procédé de fabrication d'un capuchon de pointe de seringue (20) selon l'une des revendications précédentes, le procédé comprenant les étapes suivantes:
- préparer, par un processus de moulage à chaud, un corps (21) comprenant deux faces d'extrémité (22, 23), l'une desdites faces d'extrémité (22) étant prévue d'un orifice (28) débouchant sur un alésage borne axial (24) comportant un fond (25) et une paroi latérale interne, ledit corps consistant en un matériau élastique,
- préparer, par un processus de moulage à chaud, un insert (26) consistant en un matériau élastique comportant deux faces d'extrémité, le matériau élastique du corps étant différent de celui-ci de l'insert et le matériau du corps étant conçu pour assurer une ajustage étroite et stable du capuchon sur le pointe de seringue,
- le matériau élastique étant caoutchouc naturel ou synthétique,
- revêtir au moins l'une des faces d'extrémité de l'insert (26) d'un film fluoropolymère (29),
- le film fluoropolymère (29) comprenant de polytétrafluoroéthylène, de copolymère de fluoroéthylènepropylène ou de copolymère d'éthylènetetrafluoroéthylène,
- monter le capuchon de pointe (20) en introduisant, dans l'alésage borne, ledit insert de sorte que la face d'extrémité de l'insert revêtue de fluoropolymère est exposée vers l'alésage (24) et l'autre extrémité vient en butée contre le fond (25) de l'alésage (24).

8. Procédé selon la revendication 7, dans lequel le processus de moulage à chaud est un moulage par compression, un moulage par compression ou injection par transfert, un moulage à coussin ou un moulage par injection de poudre.

9. Procédé selon l'une des revendications 7 ou 8, dans lequel on ajoute au matériau élastique un agent de durcissement, pour vulcaniser le matériau pendant le moulage à chaud.

10. Procédé selon l'une des revendications 7 à 9, dans lequel une ébauche en film fluoropolymère (29) est placée dans le moule, avant le moulage de l'insert (26).
